# EUROPEAN PATENT SPECIFICATION

(11) **EP 2 678 333 B1**
(45) Date of publication and mention of the grant of the patent: **08.04.2015**
(21) Application number: 12709178.3
(22) Date of filing: 21.02.2012
(51) Int. Cl.: C07D 403/06

(54) **AN IMPROVED PROCESS FOR THE PREPARATION OF VORICONAZOLE AND INTERMEDIATES THEREOF**
VERBESSERTES VERFAHREN ZUR HERSTELLUNG VON VORICONAZOL UND ZWISCHENPRODUKTE DAVON
PROCÉDÉ AMÉLIORÉ DE PRÉPARATION DU VORICONAZOLE ET DE SES INTERMÉDIAIRES

(30) Priority: 21.02.2011 IN DE04332011
(43) Date of publication of application: 01.01.2014
(73) Proprietor: Ranbaxy Laboratories Limited, Delhi 110019 (IN)
(72) Inventor: MAHESHWARI, Nitin, New Delhi, Delhi 110064 (IN); MEDHANE, Roshan, Ramesh, Nashik, Maharashtra 422002 (IN); PRASAD, Mohan, Gurgaon, Haryana 122003 (IN); ARORA, Sudershan, Kumar, Gurgaon, Haryana 122001 (IN)
(74) Representative: Cronin, Brian Harold John
(86) International application number: PCT/IB2012/050791
(87) International publication number: WO 2012/114273

(56) References cited:
- WO-A1-97/06160
- MIKE BUTTERS ET AL: "Process Development of Voriconazole: A Novel Broad-Spectrum Triazole Antifungal Agent", ORGANIC PROCESS RESEARCH AND DEVELOPMENT, AMERICAN CHEMICAL SOCIETY, US, vol. 5, no. 1, 21 November 2000 (2000-11-21), pages 28-36, XP002620707, ISSN: 1083-6160, DOI: 10.1021/OP0000879 [retrieved on 2001-01-19]
- PALMER M H ET AL: "Partial Asymmetric Synthesis of beta-Hydroxy-acids.Part 1. Beta-Hydroxy-beta-phenylbutyric Acid", JOURNAL OF THE CHEMICAL SOCIETY, CHEMICAL SOCIETY, LETCHWORTH; GB, no. PART 01, 1 January 1960 (1960-01-01), pages 931-938, XP002091888, ISSN: 0368-1769, DOI: 10.1039/JR9600000931

## Description

### Field of the Invention

The present invention relates to an improved stereoselective process for the preparation of (2R,3S/2S,3R)-3-(4-chloro-5-fluoropyrimidin-6-yl)-2-(2,4-difluorophenyl)-1-(1H-1,2,4-triazol-1-yl)butan-2-ol intermediate compound. This intermediate compound is further used to prepare voriconazole - a triazole antifungal agent.

### Background of the Invention

VFEND® (voriconazole) is a triazole antifungal agent and has been disclosed in European patents EP 0357241 and EP 0440372. Voriconazole, chemically is (2R,3S)-2-(2,4-difluorophenyl)-3-(5-fluoro-4-pyrimidinyl)-1-(1*H*-1,2,4-triazol-1-yl)-2-butanol and represented by following chemical Formula I.

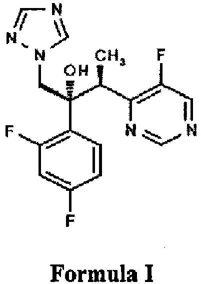

EP 0440372B1 provides a process for the preparation of (2R,3S/2S,3R)-3-(4-chloro-5-fluoropyrimidin-6-yl)-2-(2,4-difluorophenyl)-1-(1H-1,2,4-triazol-1-yl)butan-2-ol - a key intermediate for the preparation of voriconazole, comprising chromatographic separation of the two pairs of enantiomers obtained through the addition of an organolithium derivative of 4-chloro-6-ethyl-5-fluoropyrimidine to 1-(2,4-difluorophenyl)-2-(1H-1,2,4-triazol-1-yl)ethanone at from -70°C to -50°C.

EP 0871625B1 describes a stereoselective process for preparation of (2R,3S/2S,3R)-3-(4-chloro-5-fluoropyrimidin-6-yl)-2-(2,4-difluorophenyl)-1-(1H-1,2,4-triazol-1-yl)butan-2-ol or an acid addition salt thereof comprising reacting 1-(2,4-difluorophenyl)-2-(1H-1,2,4-triazol-1-yl)ethanone with 6-(1-bromoethyl)-4-chloro-5-fluoropyrimidine in the presence of zinc, iodine and/or a Lewis acid at about 0°C -5°C. It is known that the use of iodine in Reformatsky reactions works as a catalyst and decreases the rate of enolization, but is also known to cause dehydration of the product (J. Am. Chem. Soc., (1915) 37 (7), pp. 1748-1763 and J. Am. Chem. Soc., (1941) 63 (1), pp. 111-112).

The present inventors have developed an alternate stereoselective process for the preparation of (2R,3S/2S,3R)-3-(4-chloro-5-fluoropyrimidin-6-yl)-2-(2,4-difluorophenyl)-1-(1H-1,2,4-triazol-1-yl)butan-2-ol or an acid addition salt thereof, which involves simple, cost effective and uniform reaction conditions to provide the product with reproducible batch-to-batch results (*e.g.,* in yield, purity, etc.) and is thus industrially viable.

### Summary of the Invention

The present invention provides a stereoselective process for the preparation of (2R,3S/2S,3R)-3-(4-chloro-5-fluoropyrimidin-6-yl)-2-(2,4-difluorophenyl)-1-(1H-1,2,4-triazol-1-yl)butan-2-ol or an acid addition salt thereof comprising a step of reacting 6-(1-bromoethyl)-4-chloro-5-fluoropyrimidine with 1-(2,4-difluorophenyl)-2-(1H-1,2,4-triazol-1-yl)ethanone in the presence of zinc, bromine, and/or an aprotic organic solvent.

The present inventors have surprisingly found that the use of bromine is advantageous over the use of iodine in the aforementioned reaction step as bromine is not only cost effective but also provides consistent results in terms of yield and purity of the product.

The present invention also provides a process for the preparation of voriconazole using (2R,3S/2S,3R)-3-(4-chloro-5-fluoropyrimidin-6-yl)-2-(2,4-difluorophenyl)-1-(1H-1,2,4-triazol-1-yl)butan-2-ol or an acid addition salt thereof which is obtained comprising a step of reacting 6-(1-bromoethyl)-4-chloro-5-fluoropyrimidine with 1-(2,4-difluorophenyl)-2-(1H-1,2,4-triazol-1-yl)ethanone in the presence of zinc, bromine, and/or an aprotic organic solvent.

### Detailed Description of the Invention

The term "about" as used herein, in reference to parameters defined herein, such as temperature, volume, etc., refers to a variation of ± 10%. In reference to purity, the term "about", as used herein, refers to a ±5% variation.

The term "trans-enantiomeric pair" as used herein, in reference to certain compounds refers to (2R,3S/2S,3R) enantiomeric pair of the compound.

The term "cis-enantiomeric pair" as used herein, in reference to certain compounds refers to (2R,3R/2S,3S) enantiomeric pair of the compound.

A first aspect of the present invention provides a stereoselective process for the preparation of (2R,3S/2S,3R)-3-(4-chloro-5-fluoropyrimidin-6-yl)-2-(2,4-difluorophenyl)-1-(1H-1,2,4-triazol-1-yl)butan-2-ol intermediate compound of Formula II or an acid addition salt thereof 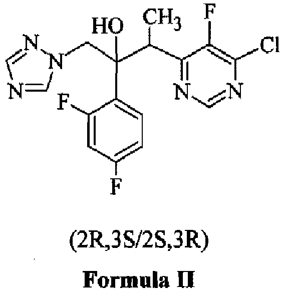 comprising a step of reacting 1-(2,4-difluorophenyl)-2-(1H-1,2,4-triazol-1-yl)ethanone compound of Formula III 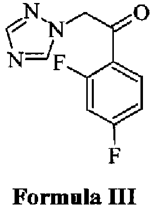 with 6-(1-bromoethyl)-4-chloro-5-fluoropyrimidine compound of Formula IV 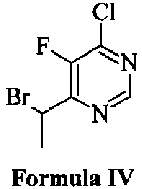 in the presence of zinc, bromine and an aprotic organic solvent.

In an embodiment of this aspect, the (2R,3S/2S,3R)-3-(4-chloro-5-fluoropyrimidin-6-yl)-2-(2,4-difluomphenyl)-1-(1H-1,2,4-triazol-1-yl)butan-2-ol intermediate compound of Formula II prepared has HPLC purity of greater than about 80%.

In another embodiment of this aspect, (2R,3S/2S,3R)-3-(4-chloro-5-fluoropyrimidin-6-yl)-2-(2,4-difluorophenyl)-1-(1H-1,2,4-triazol-1-yl)butan-2-ol intermediate compound of Formula II is prepared as hydrochloride salt thereof.

In another embodiment of this aspect, lead is also used during the reaction of the compound of Formula III with the compound of Formula IV, along with zinc.

In another embodiment of this aspect, (2R,3S/2S,3R)-3-(4-chloro-5-fluoropyrimidin-6-yl)-2-(2,4-difluorophenyl)-1-(1H-1,2,4-triazol-1-yl)butan-2-ol intermediate compound of Formula II or an acid addition salt thereof is further converted to voriconazole.

Zinc used in the above reaction may be in the form of zinc metal, dust or powder obtained from a commercial source. Optionally, lead can also be present in the reaction as metallic lead and can be added separately or be inherently present in zinc. However, it is generally preferable to add lead powder to the reaction mixture containing zinc. The mixture of zinc and lead powder, when lead is added externally, in an aprotic organic solvent is heated to a temperature from about 45°C to about 50°C followed by cooling to about 25°C -35°C. This reaction is carried out under a dry, inert atmosphere such as dry nitrogen gas.

Bromine can be used as such as commercially available or in the form of its mixture with the aprotic organic solvent and can be introduced before, after or during the addition of compound of Formula III to the reaction vessel containing zinc and/or lead in an aprotic organic solvent, wherein the temperature of the reaction mixture is maintained at about 40°C-50°C during the addition of bromine.

The aprotic organic solvent can be selected from the group consisting of ethers such as tetrahydrofuran, dioxane, 1,2-dimethoxyethane; hydrocarbons such as toluene and halohydrocarbons such as dichloromethane. The preferred solvent is tetrahydrofuran. It is preferable to dry the solvent before use to substantially remove all traces of water. Drying can be done by using any drying technique known in the art.

A solution of compound of Formula III in aprotic organic solvent can be added to the reaction mixture containing zinc, bromine and/or lead at 45°C to 50°C. The reaction mixture can then be cooled to 30°C to 40°C, followed by the addition of the compound of Formula IV. The compound of Formula IV can be added to the mixture as such, without isolation, prepared by following general procedure as exemplified in Step II of the Example 1 or in the form of isolated solid as exemplified in Example 3 of the present invention. The reaction mixture can then be cooled to about 15°C-20°C, followed by quenching with acetic acid. The resultant reaction mixture is worked up conventionally and can include filtration and washing over hyflo-bed. The solvent is then recovered under reduced pressure at a temperature of about 40-50°C to provide an oily residue. This residue is mixed with an aprotic organic solvent and then cooled from about 15°C to about 20°C followed by extraction with water and pH adjustment with inorganic acid.

The inorganic acid can be selected from hydrochloric acid, sulphuric acid, or nitric acid. Concentrated hydrochloric acid is preferably used for adjusting the pH of the mixture.

The organic layer so obtained is washed with 2%-3% ethylenediaminetetraacetic acid (EDTA) solution to remove metal impurities, if any, followed by pH adjustment using a base. The base can be selected from sodium hydroxide, potassium hydroxide, potassium carbonate, sodium carbonate, and the like. Sodium hydroxide is preferably used. An oily residue is obtained after recovery of solvent. The oily residue is treated with an organic solvent, optionally repeating the treatment, to provide the compound of Formula II, which can also be isolated as its acid addition salt. The organic solvent can be selected from the group comprising ketones, esters, and alcohols. Ketones can be selected from the group consisting of acetone, 2-butanone, 2-pentanone, 3-pentanone, methyl isobutyl ketone, and mixtures thereof. Acetone is the preferred ketone. Esters can be selected from the group consisting of ethyl acetate, n-propyl acetate and a mixture thereof. Alcohols can be selected from the group consisting of ethanol, propanol, isopropanol, butanol, pentanol, and mixtures thereof. An isopropanol solution of hydrogen chloride is preferably used to obtain the hydrochloride salt of the 'trans-enantiomeric pair' of compound of Formula II with desired purity.

The compound of Formula II as obtained by the present aspect has trans-enantiomeric (2R,3S/2S,3R) purity greater than 85%, more preferably greater than 90% and most preferably greater than 94%. The content of cis-enantiomeric pair (2R,3R/2S,3S) in compound of Formula II can vary from about 1% to 5% depending upon the batch size and scale-up conditions.

The compound of Formula II can be converted to voriconazole by following any method(s) known in prior art. For example, the method described in EP 0871625B1 can be used.

A second aspect of the present invention provides a process for the preparation of voriconazole comprising the step of reacting 1-(2,4-difluorophenyl)-2-(1H-1,2,4-triazol-1-yl)ethanone compound of Formula III 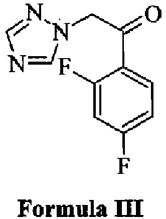 with 6-(1-bromoethyl)-4-chloro-5-fluoropyrimidine compound of Formula IV 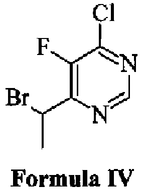 in the presence of zinc, bromine and an aprotic organic solvent and converting the product obtained thereby to voriconazole.

In an embodiment of this aspect, the product obtained by reacting the compound of Formula III with the compound of Formula IV is (2R,3S/2S,3R)-3-(4-chloro-5-fluoropyrimidin-6-yl)-2-(2,4-difluorophenyl)-1-(1H-1,2,4-triazol-1-yl)butan-2-ol represented by Formula II or an acid addition salt thereof.

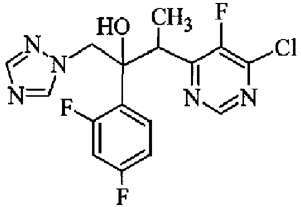

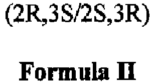

In another embodiment of this aspect, (2R,3S/2S,3R)-3-(4-chloro-5-fluoropyrimidin-6-yl)-2-(2,4-difluorophenyl)-1-(1H-1,2,4-triazol-1-yl)butan-2-ol intermediate compound of Formula II obtained has HPLC purity of about 80%.

In another embodiment of this aspect, the compound of Formula II can be isolated as the hydrochloride salt thereof.

In another embodiment of this aspect, lead can also optionally be present during reaction of the compound of Formula III with the compound of Formula IV.

The reaction conditions, reagents, methodology, etc. of the reaction between the compound of Formula III and the compound of Formula IV to obtain the compound of Formula II or an acid addition salt thereof is described in detail in the first aspect of present invention. The description provided for in the first aspect of present invention for the preparation of a compound of Formula II or an acid addition salt thereof is also applicable here in this aspect for preparing the compound of Formula II which is then converted to voriconazole.

The compound of Formula II obtained as per the present aspect can be converted to voriconazole following Example 4 of the present invention or by following any prior art method. For example, the method described in EP 0871625B1 can be used.

In another embodiment of this aspect, voriconazole having HPLC purity greater than 99.8% is obtained.

The 1-(2,4-difluorophenyl)-2-(1H-1,2,4-triazol-1-yl)ethanone compound of Formula III can be prepared by a process known in prior art, for example, EP 0440372B1 or EP 0871625B1.

In the following section, aspects and embodiments thereof are described by way of examples to illustrate the process. However, these are not intended in any way to limit the scope of the invention. Several variants of these examples would be evident to persons ordinarily skilled in the art.

### EXAMPLES

### Example 1: Preparation of Hydrochloride Salt of (2R,3S/2S,3R)-3-(4-chloro-5-fluoropyrimidin-6-yl)-2-(2,4-difluorophenyl)-1-(1H-1,2,4-triazol-1-yl)butan-2-ol

### Step I: Procedure for preparation of zinc dust

Zinc dust (500 gm) was stirred in de-ionized water (4 L) at 20°C-25°C followed by the addition of concentrated hydrochloric acid (250 mL) at the same temperature while maintaining the pH at about 1. The mixture was stirred for about 2 hours at 20°C -25°C and filtered. This was washed with de-ionized water (1500 mL + 2 x 500 mL). The resultant mixture was further washed with acetone (5 x 500 mL) and dried in a tray dryer at 110°C-120°C for about 12 hours.
Dry weight: 452 gm

### Step II: Preparation of 6-(1-Bromoethyl)-4-chloro-5-fluoropyrimidine (Formula IV)

4-Chloro-6-ethyl-5-fluoropyrimidine (187 gm) was added to dichloromethane (900 mL) and the mixture was stirred at ambient temperature under anhydrous conditions with nitrogen blanketing. To the reaction mixture azobisisobutyronitrile (10 gm) and N-Bromosuccinimide (271.2 gm) were added and stirred under reflux for 16 hours. The reaction mixture was cooled to 15°C-20°C. It was added to de-ionized water (800 mL) and sodium metabisulphite (38 gm). The reaction mixture was further stirred for about 30 minutes. The mixture was allowed to settle and layers were separated. The aqueous layer was extracted with dichloromethane (200 mL). The organic layers so obtained were combined and washed with de-ionized water (2x600 mL). The solvent was recovered under reduced pressure (50-75 torr) at 40°C-50°C and oily residue was obtained. The oily residue was used as such in step III for preparation of hydrochloride salt of (2R,3S/2S,3R)-3-(4-chloro-5-fluoropyrimidin-6-yl)-2-(2,4-difluorophenyl)-1-(1H-1,2,4-triazol-1-yl)butan-2-ol.

### Step III: Preparation of hydrochloride salt of (2R,3S/2S,3R)-3-(4-chloro-5-fluoropyrimidin-6-yl)-2-(2,4-difluorophenyl)-1-(1H-1,2,4-triazol-1-yl)butan-2-ol

A mixture of zinc dust (300 gm; from step I above) and lead powder (15.2 gm) in tetrahydrofuran (1000 mL) at ambient temperature was stirred under anhydrous condition with nitrogen blanketing. The stirred mixture was heated to 45°C-50°C and then cooled to 30°C-35°C followed by the addition of bromine (180 gm) at 45°C-50°C. The reaction mixture was stirred for 30 minutes. A solution of 1-(2,4-difluorophenyl)-2-(1H-1,2,4-triazol-1-yl)ethanone (200 gm; Formula III) in dichloromethane (1800 mL) was added to the reaction mixture at 45°C-50°C followed by cooling to 30°C-40°C. To the reaction mixture, a solution of 6-(1-bromoethyl)4-chloro-5-fluoropyrimidine (entire quantity from Step II) in dichloromethane (200 mL) was added at 30°C-40°C. This was stirred for 30 minutes to 2 hours and then cooled to 15°C-20°C. Acetic acid (300 gm) was added to it. The mixture obtained was filtered and washed with dichloromethane (2x400 mL). The solvents were recovered under reduced pressure at 45°C-50°C to provide an oily residue. To the oily residue, dichloromethane (1000 mL) was added. This mixture was stirred and cooled to 15°C-20°C followed by the addition of de-ionized water (1000 mL) and concentrated hydrochloric acid (200 mL). This was allowed to settle and the layers were separated. The organic layer was washed with 2% w/v aqueous ethylenediaminetetraacetic acid solution (EDTA solution; 1000 mL) at 15°C-20°C. De-ionized water (1000 mL) was added to the organic layer and pH was adjusted with about 40% w/v aqueous sodium hydroxide solution (98 mL) at 15°C-20°C. This was allowed to settle and the layers were separated. The solvent was recovered from the organic layer under reduced pressure (50-500 torr) at 35°C-45°C and oily residue was obtained. Acetone (400 mL) was added to the residue, stirred and then solvent was recovered under reduced pressure (50-500 torr) at 35°C-45°C. To the so obtained oily residue, acetone (1000 mL) was further added, the mixture was stirred and then cooled to 20°C-25°C. To the resultant mixture, isopropanol hydrochloride (180 gm) was added and it was stirred for about 2 hours at 20°C-25°C. The slurry so obtained was cooled to 0°C-5°C and stirred for 60 minutes at 0°C-5°C. The solid obtained was filtered and washed with acetone (600 mL) at the same temperature. The solid was dried at 40°C-50°C.
% Yield: 47.8 w.r.t compound of Formula III
% HPLC purity: 95.91%

### Example 2: Preparation of Hydrochloride Salt of (2R,3S/2S,3R)-3-(4-chloro-5-fluoropyrimidin-6-yl)-2-(2,4-difluorophenyl)-1-(1H-1,2,4-triazol-1-yl)butan-2-ol

A mixture of zinc dust (150 gm) and lead powder (7.6 gm) was stirred in tetrahydrofuran (500 mL) at ambient temperature under anhydrous conditions with nitrogen blanketing. The mixture was heated to 45°C-50°C and then cooled to 30°C-35°C followed by the addition of bromine (90 gm) at 45°C-50°C. The reaction mixture was stirred for 30 minutes. A solution of 1-(2,4-difluorophenyl)-2-(1H-1,2,4-triazol-1-yl)ethanone (100 gm) in dichloromethane (900 mL) was added to the reaction mixture at 45°C-50°C. The reaction mixture was cooled to about 30°C-35°C. To the reaction mixture, solution of 6-(1-bromoethyl)-4-chloro-5-fluoropyrimidine (entire quantity from Step II of Example 1) in dichloromethane (100 mL) was added at 30°C-40°C. This was then cooled to 15°C-20°C. Acetic acid (150 gm) was added to it. The mixture obtained was filtered and washed with dichloromethane (2 x 200 mL). The solvents were recovered under reduced pressure. The oily residue so obtained was cooled to 20°C-25°C and dichloromethane (500 mL) was added to it. The mixture was stirred and cooled to 15°C-20°C followed by addition of de-ionized water (500 mL) and concentrated hydrochloric acid (100 mL). This was allowed to settle and layers were separated. The organic layer was washed with 2% w/v aqueous ethylenediaminetetraacetic acid solution (EDTA solution; 500 mL) at 15°C-20°C. De-ionized water (500 mL) was added to the organic layer and the pH was adjusted with about 40% w/v aqueous sodium hydroxide solution (36 mL) at 15°C-20°C. This was allowed to settle and layers were separated. The solvent was recovered from the organic layer under reduced pressure at 40°C-45°C. To the resultant oily residue, ethyl acetate (200 mL) was added, stirred and then the solvent was recovered under reduced pressure. The residue so obtained was cooled to 20°C-25°C and isopropanol hydrochloride (90 gm) was added to it. Seeds of hydrochloride salt of (2R,3S/2S,3R)-3-(4-chloro-5-fluoropyrimidin-6-yl)-2-(2,4-difluorophenyl)-1-(1H-1,2,4-triazol-1-yl)butan-2-ol (1 gm) were added to the mixture and the resultant slurry was stirred at 20°C -25°C for 4 hours. The mixture was cooled to 15°C-20°C, the solid obtained was washed with ethyl acetate (3 x 200 mL). The wet material so obtained was dried at 35°C-40°C.
% yield : 48.5% w.r.t compound of Formula III
% HPLC purity: 94.37%

### Example 3: Preparation of Hydrochloride Salt of (2R,3S/2S,3R)-3-(4-chloro-5-fluoropyrimidin-6-yl)-2-(2,4-difluorophenyl)-1-(1H-1,2,4-triazol-1-yl)butan-2-ol

A mixture of zinc dust (30 gm) and lead powder (1.52 gm) was stirred in tetrahydrofuran (100 mL) at ambient temperature under anhydrous condition with nitrogen blanketing. The stirred mixture was heated to 45°C-50°C and then cooled to 30°C-35°C followed by addition of bromine (90 gm) at 45°C-50°C. The reaction mixture was stirred for 10 minutes. A solution of 1-(2,4-difluorophenyl)-2-(1H-1,2,4-triazol-1-yl)ethanone (20 gm; Formula III) in dichloromethane (180 mL) was added to the reaction mixture at 45°C-50°C followed by cooling to 30°C-35°C. The solution of 6-(1-bromoethyl)-4-chloro-5-fluoropyrimidine (27.8 gm) in dichloromethane (20 mL) was added to the reaction mixture at 30°C-35°C. This was stirred for 30 minutes to 2 hours and then cooled to 15°C-20°C. Acetic acid was added (30 gm) to it. The mixture obtained was filtered and washed with dichloromethane (2x40 mL). The solvents were recovered under reduced pressure at 40°C-45°C to provide an oily residue. This residue was cooled to 20°C-25°C. To the oily residue, dichloromethane (100 mL) was added. This mixture was stirred and cooled to 15°C-20°C followed by addition of de-ionized water (100 mL) and concentrated hydrochloric acid (20 mL). This was allowed to settle and layers were separated. The organic layer was washed with 2% w/v aqueous ethylenediaminetetraacetic acid solution (EDTA solution; 100 mL) at 15°C-20°C. De-ionized water (100 mL) was added to the organic layer and the pH was adjusted with about 40% w/v aqueous sodium hydroxide solution (8.2 mL) at 15°C-20°C. This was allowed to settle and layers were separated. The solvent was recovered from the organic layer and oily residue was obtained. Ethyl acetate (40 mL) was added to the residue, stirred and then the solvent was recovered under reduced pressure at 40°C-45°C. To the so obtained oily residue, ethyl acetate (140 mL) was further added; the mixture was stirred and then cooled to 20°C-25°C. To the resultant mixture, isopropanol hydrochloride (18 gm) was added at 20°C-25°C. Seeds of hydrochloride salt of (2R,3S/2S,3R)-3-(4-chloro-5-fluoropyrimidin-6-yl)-2-(2,4-difluorophenyl)-1-(1H-1,2,4-triazol-1-yl)butan-2-ol (0.2 gm) were added to the resultant mixture and the resultant slurry was stirred for about 66 hours at 20°C-25°C. The slurry so obtained was washed with ethyl acetate (4 x 40 mL) and the solid obtained was dried at 40°C-45°C.
% yield: 43.8% w.r.t. compound of Formula III
% HPLC purity: 79.07%

### Example 4: Preparation of (2R,3S)-3-(5-fluoropyrimidin-6-yl)-2-(2,4-difluorophenyl)-1-(1H-1,2,4-triazol-1-yl)butan-2-ol (Voriconazole)

### Step 4A: Preparation of voriconazole camphorsulfonate (wet) from hydrochloride salt of (2R,3S/2S,3R)-3-(4-chloro-5-fluoropyrimidin-6-yl)-2-(2,4-difluorophenyl)-1-(1H-1,2,4-triazol-1-yl)butan-2-ol

Hydrochloride salt of (2R,3S/2S,3R)-3-(4-chloro-5-fluoropyrimidin-6-yl)-2-(2,4-difluorophenyl)-1-(1H-1,2,4-triazol-1-yl)butan-2-ol (180 gm) obtained from Step III of Example 1 or Example 2 or Example 3 was added to methanol (720 mL) at 20°C-25°C followed by pH adjustment (8.0-9.0) with 40% w/v aqueous sodium hydroxide solution (50 mL) under nitrogen blanketing. To this reaction mixture, ammonium formate (108.2 gm) was added followed by addition of wet palladium/carbon [Pd/C (2.5 % w/w dry, 18 gm)] treated with de-ionized water. The reaction mixture was further heated and stirred at 55°C-60°C followed by cooling of the mixture to 30°C-35°C. The reaction mixture was filtered through hyflobed and washed with methanol (360 mL) first and then with a mixture of de-ionized water (1800 mL) and dichloromethane (900 mL) at 15°C-20°C followed by stirring. To the organic layer, de-ionized water (360 mL) was added followed by pH adjustment with 2N hydrochloric acid solution (6 mL) at the same temperature. The solvent was recovered under reduced pressure at 40°C-45°C to give residual solid. To the residue acetone (2700 mL) was added followed by addition of the methanolic solution of (1R)-(-)-10-camphorsulfonic acid (99.5 gm in 900 mL methanol) at 40°C-45°C. Seed of voriconazole camphorsulfonate (0.18 gm) was added to the reaction mixture and the mixture stirred at 40°C-45°C for 60 minutes. The slurry so obtained was cooled to 30°C-35°C followed by further cooling to 20°C-25°C. The mixture was stirred for 10-12 hours at 20°C-25°C followed by filtration and washing with acetone (180 mL) to give wet voriconazole camphorsulfonate.
Wet wt: 91 gm

### Step 4B: Preparation of voriconazole from voriconazole camphorsulfonate (wet)

Voriconazole camphorsulfonate (wet) (entire quantity from Step 4A of Example 4) was added to a stirred mixture of de-ionized water (360 mL) and dichloromethane (360 mL) at 15°C-25°C followed by pH adjustment of the mixture to pH 10-11 with 40% w/v aqueous sodium hydroxide solution (16.2 mL). The organic layer was washed with de-ionized water (360 mL) followed by filtration of organic layer through 0.45 micron filter. The filtered layer was further washed with dichloromethane (90 mL) and solvent was recovered under reduced pressure (50-500 torr) at 35°C-45°C to give a residual solid. Isopropyl alcohol (450 mL) was added to the residue followed by heating and stirring of the mixture at 60°C-70°C until the mixture was dissolved, followed by recovery of isopropyl alcohol under reduced pressure (50-500 torr) at 50°C-60°C to obtain a residual volume of 270 mL. The concentrated solution so obtained was heated to 60°C-70°C followed by cooling of the solution to 45°C-50°C. The solution was further cooled to 5°C-10°C followed by stirring for 60 minutes at the same temperature. The solid obtained was filtered and washed with isopropyl alcohol (135 mL) followed by drying of the solid under reduced pressure at 50°C-55°C until constant weight.
% Yield: 42.8%
% HPLC purity: 99.9%

## Claims

1. A stereoselective process for the preparation of (2R,3S/2S,3R)-3-(4-chloro-5-fluoropyrimidin-6-yl)-2-(2,4-difluorophenyl)-1-(1H-1,2,4-triazol-1-yl)butan-2-ol intermediate compound of Formula II or an acid addition salt thereof 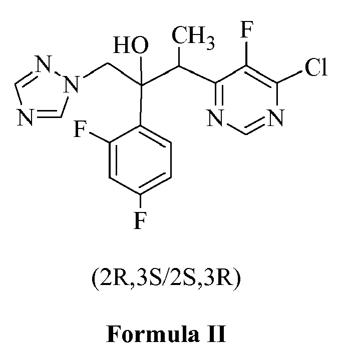 comprising the step of reacting 1-(2,4-difluorophenyl)-2-(1H-1,2,4-triazol-1-yl)ethanone compound of Formula III 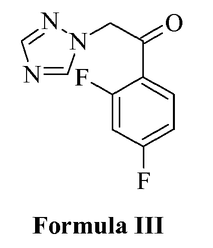 with 6-(1-bromoethyl)-4-chloro-5-fluoropyrimidine compound of Formula IV 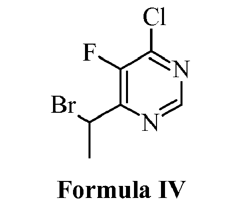 in the presence of zinc, bromine and an aprotic organic solvent.

2. The process according to claim 1, wherein the compound of Formula II is isolated as its hydrochloride salt.

3. The process according to claim 1, wherein lead is also used during the reaction of the compound of Formula III with the compound of Formula IV.

4. A process for the preparation of voriconazole of Formula I 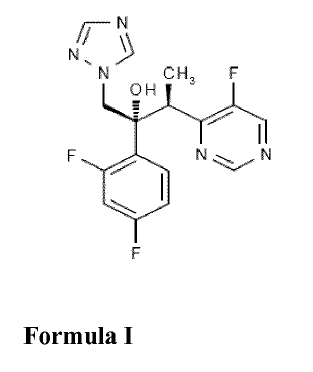 comprising preparing by the process of claim 1 a compound of Formula II or an acid addition salt thereof 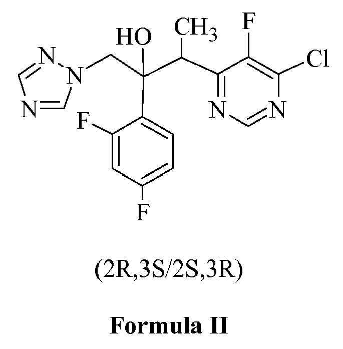 and converting the compound of Formula II or an acid addition salt thereof into voriconazole of Formula I.

5. The process according to claim 4, wherein the acid addition salt of the compound of Formula II is its hydrochloride salt.

6. The process according to claim 4, wherein lead is also used during the reaction of the compound of Formula III with the compound of Formula IV.

7. The Process of claim 4, wherein the voriconazole so prepared has an HPLC purity of greater than 99.8%.

## Patentansprüche

1. Stereoselektives Verfahren für die Herstellung der Zwischenverbindung (2R,3S/2S,3R)-3-(4-Chlor-5-fluorpyrimidin-6-yl)-2-(2,4-difluorphenyl)-1-(1H-1,2,4-triazol-1-yl)butan-2-ol nach Formel II oder eines Säureadditionssalzes davon, 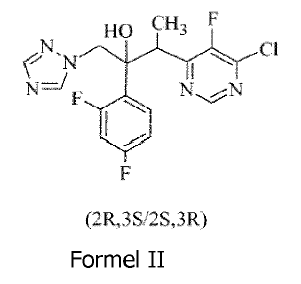 umfassend den Schritt
Umsetzen der Verbindung 1-(2,4-Difluorphenyl)-2-(1H-1,2,4-triazol-1-yl)ethanon nach Formel III 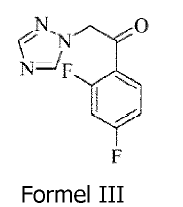 mit der Verbindung 6-(1-Bromethyl)-4-chlor-5-fluorpyrimidin nach Formel IV 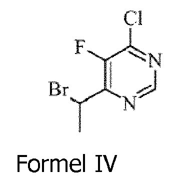 in Gegenwart von Zink, Brom und einem aprotischen organischen Lösungsmittel.

2. Verfahren nach Anspruch 1, wobei die Verbindung der Formel II als ihr Hydrochloridsalz isoliert wird.

3. Verfahren nach Anspruch 1, wobei während der Reaktion der Verbindung der Formel III mit der Verbindung der Formel IV auch Blei verwendet wird.

4. Verfahren für die Herstellung von Voriconazol der Formel I, 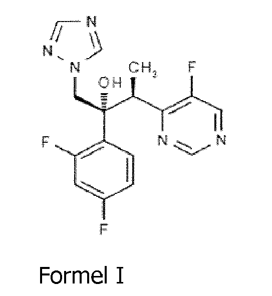 umfassend die Herstellung einer Verbindung der Formel II oder eines Säureadditionssalzes davon nach dem Verfahren gemäß Anspruch 1 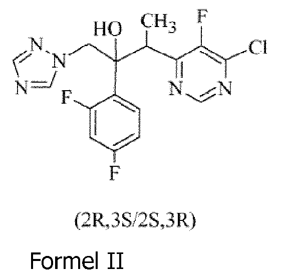 und die Umwandlung der Verbindung der Formel II oder eines Säureadditionssalzes davon in Voriconazol der Formel I.

5. Verfahren nach Anspruch 4, wobei das Säureadditionssalz der Verbindung der Formel II ihr Hydrochloridsalz ist.

6. Verfahren nach Anspruch 4, wobei während der Reaktion der Verbindung der Formel III mit der Verbindung der Formel IV auch Blei verwendet wird.

7. Verfahren nach Anspruch 4, wobei das so hergestellte Voriconazol eine HPLC-Reinheit von mehr als 99,8 % hat.

## Revendications

1. Procédé stéréosélectif pour la préparation du composé intermédiaire (2R,3S/2S,3R)-3-(4-chloro-5-fluoropyrimidin-6-yl)-2-(2,4-difluorophényl)-1-(1H-1,2,4-triazol-1-yl)butan-2-ol de formule II ou de son sel d'addition acide 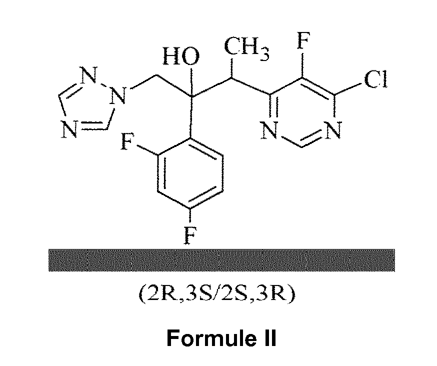 comprenant l'étape consistant à faire réagir le composé 1-(2,4-difluorophényl)-2-(1H-1,2,4-triazol-1-yl)éthanone de formule III 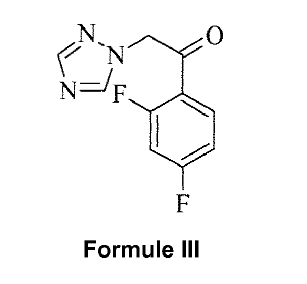 avec le composé 6-(1-bromoéthyl)-4-chloro-5-fluoropyrimidine de formule IV 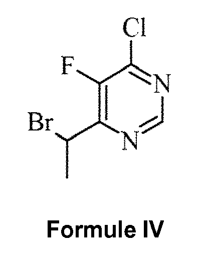 en présence de zinc, de brome et d'un solvant organique aprotique.

2. Procédé selon la revendication 1, dans lequel le composé de formule II est isolé sous la forme de son sel de chlorhydrate.

3. Procédé selon la revendication 1, dans lequel du plomb est également utilisé pendant la réaction du composé de formule III avec le composé de Formule IV.

4. Procédé de préparation de voriconazole de formule I 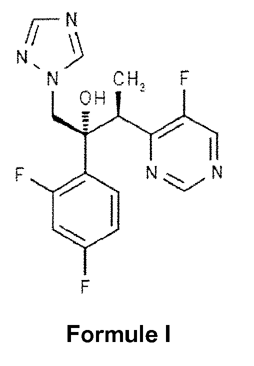 comprenant l'étape consistant à préparer par le procédé selon la revendication 1 un composé de formule II ou son sel d'addition acide 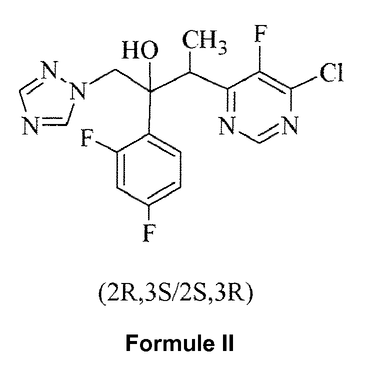 et à convertir le composé de formule II ou son sel d'addition acide en voriconazole de formule I.

5. Procédé selon la revendication 4, dans lequel le sel d'addition acide du composé de formule II est son sel de chlorhydrate.

6. Procédé selon la revendication 4, dans lequel du plomb est également utilisé pendant la réaction du composé de formule III avec le composé de formule IV.

7. Procédé selon la revendication 4, dans lequel le voriconazole ainsi préparé présente une pureté CLHP supérieure à 99,8 %.
